(19) Europäisches Patentamt
European Patent Office
Office européen des brevets

(11) EP 2 207 049 A1

(12) DEMANDE DE BREVET EUROPEEN

(43) Date de publication:
14.07.2010 Bulletin 2010/28

(51) Int Cl.:
*G01V 11/00* (2006.01)
*G01N 15/08* (2006.01)
*G06F 17/50* (2006.01)
*G01N 15/00* (2006.01)
*G01N 33/24* (2006.01)

(21) Numéro de dépôt: 09290897.9

(22) Date de dépôt: 02.12.2009

(84) Etats contractants désignés:
AT BE BG CH CY CZ DE DK EE ES FI FR GB GR
HR HU IE IS IT LI LT LU LV MC MK MT NL NO PL
PT RO SE SI SK SM TR
Etats d'extension désignés:
AL BA RS

(30) Priorité: 18.12.2008 FR 0807273

(71) Demandeur: IFP
92852 Rueil-Malmaison Cédex (FR)

(72) Inventeurs:
• Algive, Lionnel
  92500 Rueil-Malmaison (FR)
• Bekri, Samir
  92500 Rueil-Malmaison (FR)
• Vizika, Olga
  92500 Rueil-Malmaison (FR)

(54) **Méthode pour déterminer l'évolution de propriétés pétrophysiques d'une roche au cours de la diagenèse**

(57) - Méthode pour déterminer quantitativement l'évolution de perméabilité et de porosité d'un milieu poreux au cours de la diagenèse.

- On définit un scénario de diagenèse ainsi qu'une structure initiale du réseau de pores du milieu poreux. On construit une représentation du réseau de pores au moyen d'un modèle PNM. Puis, pour chaque étape du scénario de diagenèse, on réalise les étapes suivantes : on détermine la concentration en ions aux parois des pores et canaux du modèle PNM, pour une réaction de précipitation ou de dissolution en fonction du scénario, et on en déduit une variation de géométrie du modèle PNM. Pour le modèle PNM ainsi modifié, on calcule la porosité de façon géométrique et la perméabilité à partir de la loi de Darcy. On réitère ces étapes en fonction du scénario de diagenèse. Enfin, on en déduit une loi entre la perméabilité du milieu poreux et la porosité du milieu poreux au cours de la diagenèse.

- Application notamment à l'exploitation de gisements pétroliers.

Fig. 1

EP 2 207 049 A1

## Description

La présente invention concerne le domaine de l'exploration et la production de champs pétroliers. L'invention concerne notamment une méthode pour prendre en compte l'évolution des propriétés pétrophysiques au cours de la diagenèse, pour l'étude des écoulements de fluides au sein d'une formation hétérogène. La méthode permet ainsi de déterminer l'emplacement potentiel de réservoir souterrain au sein d'un bassin sédimentaire, ou d'améliorer la récupération des hydrocarbures dans un gisement, ou réservoir souterrain.

La diagenèse regroupe l'ensemble des mécanismes physico-chimiques responsables de la transformation des sédiments en roches sédimentaires. Lors de la diagenèse, une partie des sédiments est dissoute puis transportée. Au cours du transport, le changement des conditions thermodynamiques cause la précipitation des ions conduisant à la cimentation des sédiments et à la formation de la roche (lithification). Ces changements thermodynamiques sont dus, soit à des variations des propriétés physiques (pression, température), soit à des modifications de la composition chimique (mélange avec d'autres espèces dissoutes). Les minéraux peuvent être ensuite redissous puis cristallisés à nouveau. L'alternance de ces cycles de dissolution et de précipitation conduit à l'évolution progressive du milieu.

La diagenèse est donc un processus transformant un milieu poreux granulaire homogène en un milieu consolidé hétérogène. Les propriétés pétrophysiques des roches sédimentaires obtenues dépendent étroitement du cycle diagénétique qui modifie les porosités et perméabilités initiales. Le développement inégal de la diagenèse en temps et en espace est responsable des hétérogénéités constatées tant à l'échelle locale qu'à l'échelle du bassin sédimentaire.

Une meilleure compréhension de ces phénomènes permet donc d'extrapoler plus sûrement les caractéristiques des roches à partir des échantillons qui ont pu être prélevés et analysés.

Appliquées au domaine pétrolier, ces informations conduisent à un meilleur développement du champ en améliorant la caractérisation du gisement. D'une part, les réserves peuvent être évaluées plus précisément s'il est possible d'estimer l'évolution de la porosité due à la diagenèse, qui a un impact direct sur la quantité d'hydrocarbures potentiellement accumulée. D'autre part, le plan de production peut être adapté aux perméabilités estimées en optimisant pour le mieux les facilités d'extractions. Ainsi, la reconstruction du cycle diagénétique est un moyen de mieux caractériser les hétérogénéités, et fournit donc une aide appréciable lors de l'élaboration du scénario de production.

L'industrie pétrolière a donc un besoin d'outils permettant la modélisation pétrophysique de la diagenèse. Il s'agit de déterminer l'évolution au cours du temps des propriétés pétrophysiques des roches, et en particulier la perméabilité et la porosité, suite aux cycles de dissolution-précipitation de la diagenèse.

## État de la technique

Actuellement, il n'existe pas de méthodes donnant l'évolution des perméabilités et des porosités au cours de la diagenèse. Néanmoins, pour un faciès, c'est-à-dire un type de roche donné plus ou moins associé à une histoire diagénétique particulière, les géologues résument leurs observations dans des corrélations empiriques. Une telle approche est illustrée dans l'étude de Lucia, F. Jerry, "Carbonate Reservoir Characterization", Springer, (2007), EAN13: 9783540727408.

Par ailleurs les pétrophysiciens ont établi des modèles pour relier la perméabilité à la porosité. Un des plus célèbres reste les lois de Kozeny-Carman (Carman P. C., Fluid flow through granular bed, Trans. Inst. Chem. Eng. Lond., 1937, 15, p. 150-166). Toutefois, ces corrélations ne sont valides qu'à un instant précis, toujours pour un type de structure (faciès) donné. Or, au cours de la diagenèse, la structure est modifiée et le milieu poreux peut suivre une relation perméabilité-porosité différente. A ce jour, on ne sait pas quantifier leur modification respective.

Ainsi, l'objet de l'invention concerne une méthode pour suivre l'évolution des propriétés pétrophysiques d'un milieu poreux au cours de la diagenèse. Elle se base sur une étude à l'échelle des pores, au moyen d'une modélisation du réseau de pore du milieu poreux (roche) dont la géométrie varie au cours de la diagenèse.

## La méthode selon l'invention

L'invention concerne une méthode pour déterminer quantitativement une évolution de perméabilité et de porosité d'un milieu poreux au cours d'une diagenèse, le milieu poreux comportant un réseau de pores. La méthode comporte les étapes suivantes :

- on définit un scénario de diagenèse comportant un ensemble d'étapes de précipitation et de dissolution dans le milieu poreux, ainsi qu'une structure initiale du réseau de pores, au moyen de mesures physiques et d'observations dudit milieu ;

- on construit une représentation du réseau de pores, au moyen d'un modèle PNM comportant un ensemble de noeuds de géométrie connue reliés par des canaux de géométrie connue ; puis, pour chaque étape dudit scénario de diagenèse, on réalise les étapes *a* à *d* suivantes :

a. on détermine une concentration en ions aux parois de chaque noeud et canal ;

b. on en déduit une variation de géométrie des noeuds et canaux dudit modèle PNM ;

c. on détermine une perméabilité et une porosité dudit modèle PNM ainsi modifiées ;

d. on réitère les étapes *a* à *c* jusqu'à ce que ladite étape soit achevée conformément au scénario de diagenèse ; et

- on détermine une loi entre la perméabilité du milieu poreux et la porosité du milieu poreux au cours de la diagenèse.

Selon l'invention, on peut construire la représentation du réseau de pores au moyen d'expériences d'invasion de mercure sur des carottes extraites du milieu poreux. On peut également déterminer à la fin de chaque étape du scénario de diagenèse d'autres propriétés pétrophysiques, telles que pression capillaire et perméabilités relatives.

Selon l'invention, on peut déterminer la porosité au moyen de calculs de volumes connaissant la géométrie du modèle PNM, et on peut déterminer la perméabilité en utilisant la loi de Darcy.

L'invention concerne également une méthode pour déterminer l'emplacement potentiel d'un réservoir souterrain au sein d'un bassin sédimentaire constituant un milieu poreux. Selon cette méthode on détermine une loi entre la perméabilité du milieu poreux et la porosité du milieu poreux au cours d'un phénomène de diagenèse subit par le bassin au moyen de la méthode selon l'invention. Puis, on étudie des écoulements de fluides au sein du bassin au moyen d'un simulateur de bassin renseigné par cette loi.

L'invention concerne également une méthode pour améliorer une récupération d'hydrocarbures dans un réservoir souterrain constituant un milieu poreux, dans laquelle on caractérise des hétérogénéités dudit réservoir en déterminant une loi entre la perméabilité et la porosité du réservoir au cours d'un phénomène de diagenèse subit par le réservoir au moyen de la méthode selon l'invention, et on étudie des écoulements de fluides au sein du réservoir au moyen d'un simulateur de réservoir renseigné par cette loi.

D'autres caractéristiques et avantages de la méthode selon l'invention, apparaîtront à la lecture de la description ci-après d'exemples non limitatifs de réalisations, en se référant aux figures annexées et décrites ci-après.

**Présentation succincte des figures**

- la figure 1 représente les étapes de la méthode selon l'invention pour étudier la diagenèse d'un point de vue pétrophysique à l'échelle du pore.

- la figure 2 schématise une cellule-unité d'un modèle "réseau de pores" avec un noeud cubique (corps du pore) et six canaux à sections triangulaires (gorges ou seuils du pore).

- la figure 3 montre un cycle diagénétique dans un milieu homogène et pour lequel la vitesse intrinsèque de dissolution égale celle de précipitation.

- la figure 4 illustre un cycle diagénétique dans un milieu homogène mais pour lequel la vitesse intrinsèque de dissolution est 100 fois plus lente que celle de précipitation.

- la figure 5 montre un cycle diagénétique dans un milieu hétérogène et pour lequel la vitesse intrinsèque de dissolution est 100 fois plus lente que celle de précipitation. La présence d'hétérogénéité inverse le sens global d'évolution de la perméabilité.

- la figure 6 représente le champ de concentration en soluté constaté dans le réseau pour le cycle diagénétique de la figure 5 lors de la précipitation. Les fortes concentrations (en noire) sont généralement présentes dans les grands pores, ce qui explique la chute plus prononcée de la porosité sur la figure 5 par rapport à la figure 4.

- la figure 7 illustre, pour le cycle diagénétique de la figure 5, la translation de la distribution de tailles de pores lors de la dissolution. On assiste à une simple translation en raison de la lenteur de la réaction par rapport au transport diffusif ce qui permet d'homogénéiser la concentration en soluté.

**Description détaillée de la méthode**

L'invention concerne un procédé pour déterminer l'évolution des propriétés pétrophysiques de roches au cours de la diagenèse. Cette information peut être utilisée par un simulateur de bassin et/ou un simulateur de réservoir dans le cadre de l'exploration - production pétrolière.

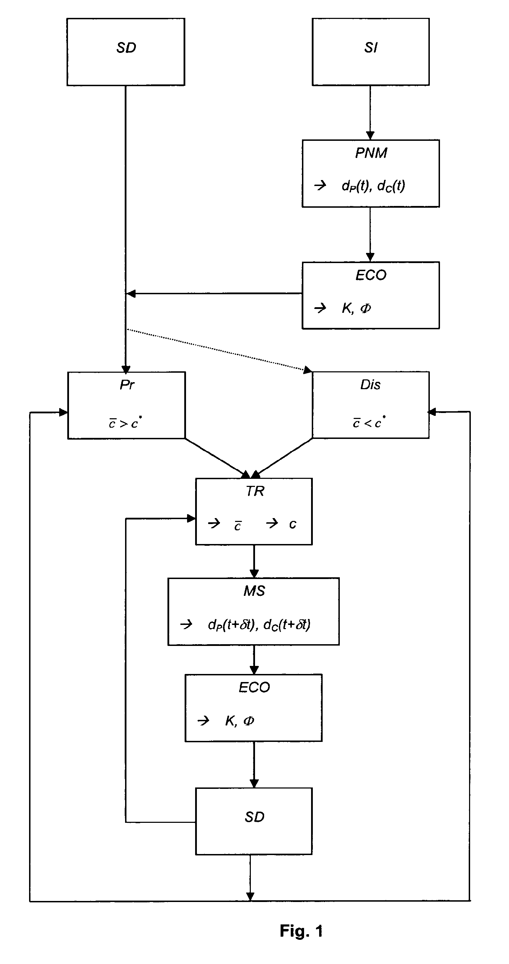

La figure 1 illustre les différentes étapes de ce procédé qui comporte :

A. Détermination d'un scénario de diagenèse (*SD*).

B. Détermination de l'évolution des propriétés pétrophysiques au cours de la diagenèse.

1. Construction d'un modèle du réseau poreux (*PNM*)

2. Détermination de l'évolution de la porosité et de la perméabilité

2a. Détermination de la porosité ($\Phi$) et de la perméabilité (K) initiale : *ECO*

2b. Détermination des concentrations globales ($\bar{c}$) et locales (c) : *TR*

2c. Détermination des modifications de structure du réseau poreux : *MS*

2d. Détermination de la porosité et de la perméabilité après réaction : *ECO*

En suivant le scénario de diagenèse défini à l'étape A, les étapes 2a à 2d sont successivement exécutées pour une réaction de précipitation (*Pr*), puis pour une réaction de dissolution (*Dis*), comme l'illustre la figure 1.

A. Détermination d'un scénario de diagenèse.

Suite à des études de terrains (études géologiques, géophysiques, pétrophysiques,...), on détermine la date de formation d'un bassin sédimentaire : il y a 10 millions d'années par exemple. Par analogie avec le présent, base de la science géologique qui suppose que les mêmes causes conduiront aux mêmes effets, on est à même de définir la structure de ce milieu poreux. On parle alors de structure initiale (*SI*). Ce milieu va ensuite subir les effets de la diagenèse et se transformer en roche. Pour évaluer ces effets, on doit définir un scénario de diagenèse. Il s'agit de définir la chronologie des alternances des cycles de précipitation et de dissolution. On va par exemple considérer que la roche a subi pendant les 200000 premières années suivant sa mise en place, des précipitations, puis des dissolutions pendant un million d'années, puis à nouveau des précipitations pendant deux millions d'années, puis...

A ce stade, ce scénario diagénétique ne permet de prédire l'évolution des propriétés pétrophysiques au cours de la diagenèse que qualitativement, à savoir hausse ou baisse de la perméabilité ou de la porosité. La quantification de ces évolutions fait l'objet du point B.

B. Détermination de l'évolution des propriétés pétrophysiques au cours de la diagenèse.

*1. Construction d'un modèle du réseau poreux*

Selon l'invention on modélise le phénomène de diagenèse, c'est-à-dire les phénomènes de transport des ions, leur dissolution et leur précipitation, à l'échelle du pore. Pour ce faire, on utilise une représentation spatiale simplifiée du réseau poreux que forment les pores de la roche.

Pour ce faire, on utilise un type de représentation bien connue des spécialistes, appelé « Pore Network Modelling » (PNM). Une description détaillée de cette technique de modélisation du réseau de pores (PNM) en termes d'approche, de caractéristiques du modèle et de sa construction, est présentée dans le document suivant :

Laroche, C. and Vizika, O., "Two-phase flow properties prediction from small-scale data using pore-network modeling", Transport in Porous Media, (2005), 61, 1, 77-91.

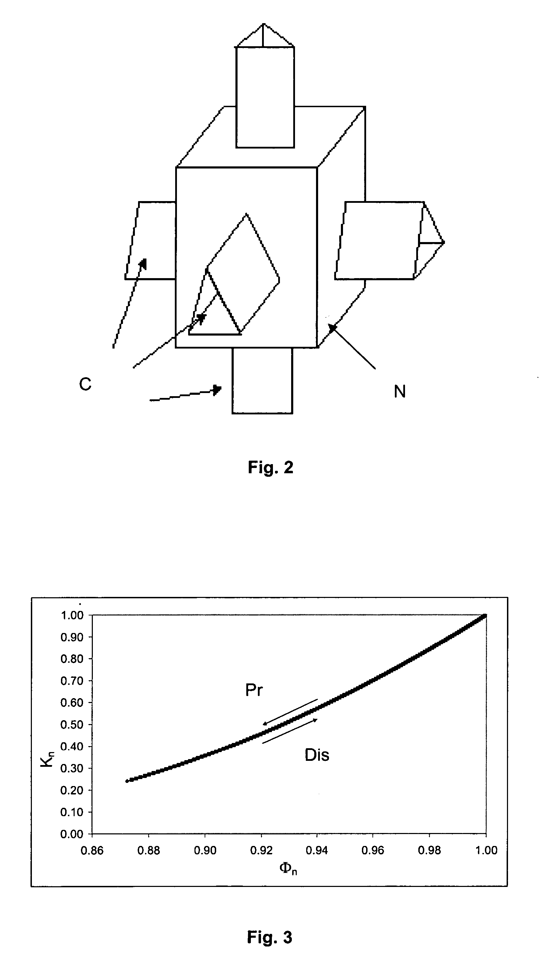

Ce modèle du réseau de pores est une représentation conceptuelle d'un milieu poreux, dont le but est de rendre compte de la physique des écoulements et des phénomènes de transport, sans s'attacher à la structure réelle du réseau formé par les pores du milieu poreux (roche). La structure y est modélisée par un réseau tridimensionnel de pores, constituant les noeuds, interconnectés par des canaux, représentant les liens entre les pores. Bien qu'il ne décrive pas la morphologie exacte du milieu poreux, un tel modèle est capable de prendre en compte les caractéristiques essentielles de topologie et de morphologie de l'espace poreux. Un milieu poreux réel comporte des angulosités et des recoins qui favorisent l'écoulement du fluide mouillant, même lorsque le centre du canal ou du pore est rempli par un fluide non mouillant. Pour rendre compte de ce fait, qui influe sur la récupération, il est préférable de considérer des sections angulaires pour les pores et les canaux. Le réseau de pores est donc représenté par une matrice cubique tridimensionnelle de pores, interconnectés entre eux par des canaux, et possédant, généralement, un nombre de coordination de six (mais il peut être variable), ce qui signifie que 6 canaux sont connectés à chaque pore. On appelle cellule unité, ou maille, du modèle de réseau, un noeud (N) et ses canaux (C), comme illustré sur la figure 2.

Pour construire un tel modèle, il est nécessaire de procéder en laboratoire à des expériences d'invasion de mercure (« porosimétrie mercure »). Cette technique, bien connue des spécialistes, permet de déterminer les distributions de tailles des seuils, représentés par les canaux dans le modèle réseau (PNM).

A partir de cette distribution, on détermine la distribution de taille de pores. Pour cela on considère une corrélation entre les pores et leurs canaux adjacents. On fixe alors un rapport d'aspect (AR) reliant le diamètre de pore $d_p$ à celui du canal $d_c$. Lors de la construction du réseau, les diamètres des canaux sont assignés de manière aléatoire en accord avec la distribution expérimentale obtenue par porosimétrie mercure. Il faut noter que dans le cas d'une section triangulaire, le diamètre correspond à celui du cercle inscrit dans le triangle considéré.

*2. Détermination de l'évolution de la porosité et de la perméabilité*

Le modèle du réseau de pore (PNM) permet alors de décrire les effets d'un écoulement réactif sur les propriétés du transport et sur l'évolution de la structure.

On utilise une approche numérique pour simuler l'évolution des propriétés pétrophysiques causée par l'alternance de dissolutions et précipitations. D'un point de vue pétrophysique, l'étude de la diagenèse s'articule autour de deux tâches : la résolution du transport réactif, qui consiste à déterminer le champ de concentration dans le réseau de pore, et le calcul des modifications de structure potentiellement causées par les réactions.

Ces deux aspects de la diagenèse sont résolus séparément : le procédé selon l'invention est un procédé dit « pas à pas » : la partie transport (écoulement inclus) est résolue à géométrie constante, et les modifications de structure des pores sont déterminées à concentrations constantes.

## 2a. Détermination de la porosité et de la perméabilité initiale

On peut alors déterminer la porosité du réseau poreux, correspondant à la porosité à l'échelle de la carotte. En effet la porosité se définit comme le rapport entre le volume des vides sur le volume total. Le volume total du réseau de pores est connu (Lx*Ly*Lz, produit des longueurs du réseau de pores dans chaque direction), et le volume des vides correspond aux volumes des pores et aux volumes des canaux. Ces volumes s'obtiennent par de simples calculs géométriques (volume d'un cylindre, d'une sphère,...).

La détermination de l'écoulement est un préalable à toute étude de transport en présence de convection. Il s'agit, pour une structure initiale de roche donnée, de déterminer le champ de pression. Pour chaque canal d'une cellule unité du modèle de réseau poreux (PNM), on calcule les conductances à partir de la solution bien connue de Poiseuille pour un écoulement laminaire. Ces conductances relient de manière linéaire le débit et la différence de pressions entre deux noeuds adjacents.

$$Q_{ij} = g_{ij}\left(P_i - P_j\right)$$

où : $Q_{ij}$ est le débit entre les pores $i$ et $j$

$g_{ij}$ est la conductance hydraulique du canal entre les noeuds $i$ et $j$

$P_i$ et $P_j$ sont respectivement les pressions du noeud $i$ et du noeud $j$.

On écrit ensuite la conservation des débits au noeud. On a donc $n$ équations, à sept inconnues chacune, si on suppose un réseau de $n$ pores ayant un nombre de coordination égal à 6.

$$\sum_{j=1}^{6} Q_{ij} = \sum_{j=1}^{6} g_{ij}\left(P_i - P_j\right) = 0$$

Ce système linéaire peut être synthétisé sous la forme matricielle suivante :

$$\mathbf{Ax} = \mathbf{b}$$

où : **A** est la matrice contenant les conductances

**x** est le vecteur inconnu composé des $n$ pressions

**b** est le vecteur second membre contenant les conditions aux limites

Les $n$ pressions inconnues sont alors déterminées par des méthodes classiques de résolution comme par exemple la méthode du gradient biconjugué.

Connaissant les pressions, on peut ensuite, à l'aide des conductances, calculer les débits puis les vitesses dans chaque canal.

A l'échelle du réseau, on en déduira la perméabilité de l'équation de Darcy, reliant le débit total au gradient de pression.

Une description détaillée de ces techniques de détermination de la perméabilité et de la porosité, est présentée dans le document suivant : Laroche, C. and Vizika, O., "Two-phase flow properties prediction from small-scale data using pore-network modeling", Transport in Porous Media, (2005), 61, 1, 77-91.

**2b. Détermination des concentrations en ions**

La résolution du transport réactif, consiste à résoudre, sur l'ensemble du PNM, l'équation macroscopique de convection-dispersion pour un soluté réactif en présence de réaction (précipitation, dissolution). En supposant une loi cinétique linéaire (mais la méthodologie peut être appliquée à des réactions plus complexes) cette équation s'écrit :

$$\frac{\partial \overline{c}}{\partial t} + \nabla \cdot \left( \overline{\mathbf{v}}^* \overline{c} - \overline{\overline{\mathbf{D}}}^* \nabla \overline{c} \right) + \overline{\gamma}^* \left( \overline{c} - c^* \right) = 0$$

où :

$\overline{c}$ est la concentration moyenne d'une cellule-unité du réseau

$c^*$ est la concentration d'équilibre

$\gamma^*$ est le coefficient réactif apparent, dérivant de réactions volumiques et/ou surfaciques

$\overline{\mathbf{v}}^*$ est la vitesse moyenne du soluté, différente de la vitesse moyenne du fluide

$\overline{\overline{\mathbf{D}}}^*$ est le coefficient de dispersion, ou tenseur dispersif du soluté (non réduit à la dispersion de Taylor-Aris).

Les coefficients $\overline{\gamma}^*$, $\overline{\mathbf{v}}^*$ et $\overline{\overline{\mathbf{D}}}^*$ sont appelés coefficients macroscopiques. Ces coefficients sont calculés analytiquement pour chaque cellule-unité du réseau, en résolvant les équations microscopiques et en effectuant un changement d'échelle. Il est alors possible de déterminer la carte des dépôts, puis d'en déduire leur impact sur les propriétés pétrophysiques.

Le champ de concentration $\overline{c}$ est le vecteur inconnu du système que l'on cherche à résoudre, en intégrant l'équation de conservation au noeud (bilan de masse). Ces bilans font intervenir les flux de matière (ions) entre pores, qui peuvent être exprimés en fonctions des concentrations moyennes aux noeuds et des coefficients macroscopiques du transport.

La première étape consiste donc à calculer les coefficients macroscopiques précédents pour chaque cellule-unité du réseau. Pour ce faire, on peut utiliser la méthode analytique de la théorie des moments et résoudre le problème aux valeurs propres associé. Cette technique est décrite par exemple dans le document suivant :

Shapiro M., Brenner H., Dispersion of a chemically reactive solute in a spatially model of a porous medium, Chemical Engineering Science, 1988, 43, p. 551-571.

Cette théorie repose sur l'intégration, sur un milieu supposé infini ou périodique, de l'équation macroscopique précédente pondérée par les positions. Autrement dit, on calcule les moments spatiaux. Ces moments sont comparés à ceux calculés à partir du système d'équations locales, présenté ci-après, et permettant de calculer la concentration locale, c, c'est-à-dire la concentration au sein d'un pore ou d'un canal en fonction de sa distance au centre. Ce système d'équations possède une solution analytique pour des géométries élémentaires, comme celles utilisées dans la construction du modèle PNM. On peut par exemple utiliser la technique décrite dans le document suivant pour résoudre analytiquement ce système :

Bekri S., Thovert J.-F., Adler P.M., "Dissolution of porous media", Chem. Eng. Sci., (1995) 50, 17, p. 2765-2791.

Par identification, il est alors possible d'exprimer les coefficients macroscopiques à l'aide des paramètres locaux (constante cinétique à la paroi, vitesses locales du fluide, diffusion moléculaire...).

$$\left\{ \begin{array}{l} \dfrac{\partial c}{\partial t} + \nabla \cdot \left( \mathbf{v}c - D\nabla c \right) = 0 \\ \left( \mathbf{v}c - D\nabla c \right)\mathbf{n} = \kappa c \quad sur \quad S_p \end{array} \right.$$

où

$D$ est le coefficient de diffusion moléculaire,

$\mathbf{n}$ est la normale à la paroi pointant vers le solide,

$k$ est la constante de vitesse de réaction.

$S_p$ : Surface de la paroi

Au cours de la seconde étape, connaissant explicitement ces coefficients, l'équation aux dérivées partielles du transport macroscopique, qui se résume à une équation différentielle ordinaire en régime asymptotique, est résolue analytiquement dans un canal. Ayant déterminé les concentrations moyennes le long de l'axe du canal, on déduit les flux de matière entrant dans chaque pore. Ce calcul permet d'estimer les flux avec une précision inégalée par les approximations numériques ordinaires, type schéma amont pour la convection et approximation linéaire pour la diffusion.

Enfin, au cours de la troisième étape, on écrit le système d'équation sous forme matricielle. L'équation matricielle est ensuite résolue par inversion pour obtenir le champ de concentrations. Le champ de concentration à l'échelle du réseau (carotte) est ainsi obtenu à partir d'un calcul des flux d'ions à l'échelle des pores.

### 2c. Détermination des modifications de structure du réseau poreux

Les modifications structurales du réseau de pores correspondent à une modification du diamètre des pores et/ou des canaux suite aux réactions de précipitation et dissolution.

On a déterminé au cours de l'étape 2.b les concentrations moyennes en ions, ainsi que la concentration à la paroi (c au niveau de $S_p$). Ayant mesuré expérimentalement la cinétique intrinsèque $\kappa$ de la réaction étudiée, par exemple la dissolution de la calcite, on calcule, à partir de cette concentration à l'interface, $\varphi_i$ la densité de flux réactif d'ions émis ou consommés.

$$\varphi_i = \kappa \left( c - c^* \right)$$

Connaissant la stoechiométrie de la réaction, la masse molaire et la masse volumique du minéral formé, on relie ces flux à une couche infinitésimale de minéral crée ou ôtée, et donc à un taux d'accroissement relatif du pore. Évidemment, cette couche n'est pas nécessairement uniforme. Sa distribution dans le réseau dépend des régimes de réaction et d'écoulement.

$$\left. \begin{array}{l} \varphi_m = \alpha \varphi_i \\ \\ \dfrac{\partial d}{\partial t} = \dfrac{M}{\rho} \varphi_m \end{array} \right\} \quad \Rightarrow \quad d(t + \delta t) = d(t) + \delta t \cdot \alpha \dfrac{M}{\rho} \kappa \left( c - c^* \right)$$

où :

$\alpha$ est le coefficient stoechiométrique

$\varphi_m$ la densité de flux de minéral en mol.m$^{-2}$.s$^{-1}$

$M$ et $\rho$ sont respectivement la masse molaire et la masse volumique du minéral

$d$ représente le diamètre d'un pore ou d'un canal. Ainsi $d(t)$ est le diamètre d'un pore ou d'un canal au temps $t$, et $d(t+\delta t)$ correspond au diamètre de ce pore ou de ce canal au temps $t+\delta t$.

Le temps de déformation $\delta t$ à appliquer est optimisé selon les précisons souhaitées sur l'intensité des variations de perméabilité et de porosité.

## 2d. Détermination de la porosité et de la perméabilité après réaction

Après chaque étape de déformation (étape 2c), les propriétés pétrophysiques sont recalculées, comme à l'étape 2a.

En suivant le scénario de diagenèse (*SD*) défini à l'étape A, les étapes 2a à 2d sont successivement exécutées pour une réaction de précipitation, puis pour une réaction de dissolution, comme l'illustrent les figures 1 et 3.

Outre l'intérêt d'observer la diagenèse à l'échelle du pore en dressant des cartes de dépôt du réseau, la méthode offre la possibilité de mémoriser, après chaque modification structurale, les nouvelles porosités et perméabilités en vue d'obtenir différentes corrélations. L'évolution des perméabilités et des porosités peut être utilisée par un simulateur de bassin et/ou un simulateur de réservoir dans le cadre l'exploration - production pétrolière. Ces corrélations sont intégrées dans les simulateurs de réservoir ou de bassin, en tant que données d'entrée lors de la reconstruction de l'histoire géologique du champ.

Dans le domaine pétrolier, la connaissance du cycle diagénétique peut conduire à un meilleur développement du champ, en raison de la meilleure caractérisation passée et présente du gisement. D'une part, les réserves peuvent être évaluées plus précisément en estimant l'évolution de la porosité due à la diagenèse, ce qui a un impact direct sur la quantité d'hydrocarbures potentiellement accumulée. D'autre part, le plan de production du réservoir peut être adapté aux perméabilités estimées en optimisant pour le mieux les facilités d'extractions. Ainsi, la reconstruction du cycle diagénétique est un moyen de mieux caractériser les hétérogénéités, et fournit donc une aide appréciable lors de l'élaboration du scénario de production.

La méthode permet ainsi de déterminer l'emplacement potentiel de réservoirs souterrains au sein d'un bassin sédimentaire (utilisation d'un simulateur de bassin), ou d'améliorer la récupération des hydrocarbures dans un gisement, ou réservoir souterrain (utilisation d'un simulateur de réservoir).

## Applications

On applique ci-après la méthode selon l'invention sur trois exemples différents, extrêmement simplifiés. Ces exemples permettent d'illustrer la capacité de la méthode à décrire et à interpréter les conséquences de la diagenèse sur les propriétés pétrophysiques.

Dans les exemples ci-après, les perméabilités et les porosités sont normalisées par leur valeur initiale, c'est-à-dire leur valeur avant diagenèse. Les perméabilités normalisées sont notées $K_n$, et les porosités normalisées sont notées $\Phi_n$. De plus, dans chacun de ces exemples, on choisit un scénario de diagenèse comportant, deux fois, une étape de précipitation suivie d'une étape de dissolution, dont les durées ont été fixées arbitrairement. En réalité, la durée doit coïncider avec le scénario diagénétique établi par le géologue. On considère qu'il n'y a aucun apport de matière extérieure, et qu'à la fin de la période de dissolution, la totalité du soluté préalablement précipité a été dissoute. Par conséquent, à la fin du cycle, la porosité est égale à la porosité initiale (en supposant que les cristaux formés ou enlevés aient le même volume massique). Cependant, cela ne signifie nullement que l'on retrouve la distribution de taille de pores initiale, d'où la modification probable de la perméabilité. En effet, la perméabilité est liée au diamètre des restrictions (canaux) entre pores. Or, selon les régimes, il se peut que de la matière dissoute reprécipite préférentiellement soit dans les seuils (canaux) soit dans les pores, ce qui conduit, respectivement, à une baisse ou à une hausse de perméabilité.

Les cycles diagénétiques observés sont différents selon les régimes hydrodynamiques et réactionnels. C'est pourquoi, afin d'être en mesure de comparer les expériences, on introduit succinctement les nombres adimensionnels gouvernant le transport réactif (bien connu des spécialistes), à savoir :

- le nombre de Péclet, noté *Pe*, qui compare les flux convectifs aux flux diffusifs, et

- le nombre de Péclet-Damköhler, écrit PeDa, qui confronte la vitesse de réaction à la vitesse d'acheminement du soluté vers la paroi.

Pour chaque exemple, on applique la méthode pour déterminer l'évolution de la porosité ($\phi_n$) et de la perméabilité ($K_n$) au cours de la diagenèse.

*Exemple 1 : Géométrie initiale homogène (tous les pores ont le même diamètre)*

Selon cet exemple, on considère un réseau homogène tridimensionnel de 250 pores (10*5*5). Les régimes réactionnels des précipitations et des dissolutions sont les mêmes: Pe=10, PeDa = 0,1 pour les précipitations et les dissolutions.

Dans ce cas de figure, il n'y a pas d'évolution de perméabilité. Les conditions de porosité et de perméabilité initiales et finales sont les mêmes. Pour constater finalement une évolution de perméabilité, il faut que la dissolution (Dis) et la précipitation (Pr) aient un régime réactionnel différent. Sinon, l'une annule simplement les effets de l'autre, comme l'illustre la figure 3. Cette figure montre la perméabilité ($K_n$) en fonction de la porosité ($\Phi_n$) pour un cycle diagénétique simulé dans un réseau homogène tridimensionnel de 250 pores (10*5*5), avec *Pe*=10, *PeDa* = 0,1 pour les précipitations et les dissolutions.

*Exemple 2 : Géométrie initiale homogène (tous les pores ont le même diamètre)*

Selon cet exemple, on considère toujours un réseau homogène tridimensionnel de 250 pores (10*5*5). Mais cette fois, les régimes réactionnels des précipitations et des dissolutions sont différents : *PeDa* = 0,01 pour les dissolutions, et *PeDa* = 1 pour les précipitations. Ceci correspond à une dissolution cent fois plus lente que la précipitation.

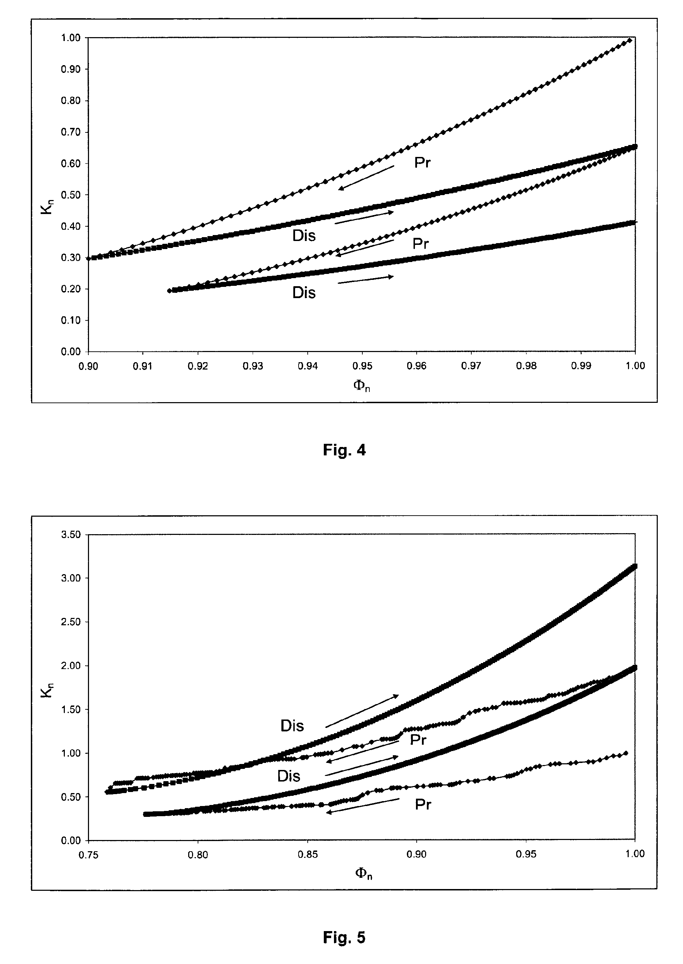

La méthode fournit l'évolution de la perméabilité et de la porosité calculées à l'échelle du réseau. La figure 4 montre la perméabilité ($K_n$) en fonction de la porosité ($\Phi_n$) pour le cycle diagénétique dans le réseau homogène tridimensionnel de 250 pores (10*5*5). On constate une chute prononcée de perméabilité au cours de la diagenèse. Ceci s'explique par l'agrandissement des pores et la réduction des canaux.

Puisque la précipitation et la dissolution ne causent pas la même déformation, à cause de régimes réactifs différents, le cycle diagénétique conduit à l'accentuation de l'hétérogénéité entre pores et canaux.

*Exemple 3 : Géométrie initiale hétérogène (tous les pores n'ont pas le même diamètre)*

Un des avantages de la méthode selon l'invention consiste aussi à prendre facilement en considération l'effet de la structure du réseau de pores. Pour illustrer cette capacité, on simule la diagenèse dans un réseau de pores plus réaliste, présentant une distribution de taille de pores.

Selon cet exemple, on choisit des régimes réactifs moyens identiques aux cas précédents : *Pe*=10, *PeDa*=1 pour la précipitation et *PeDa*=0,01 pour la dissolution. Le caractère hétéroclite des diamètres génère une hétérogénéité au sein du régime réactionnel.

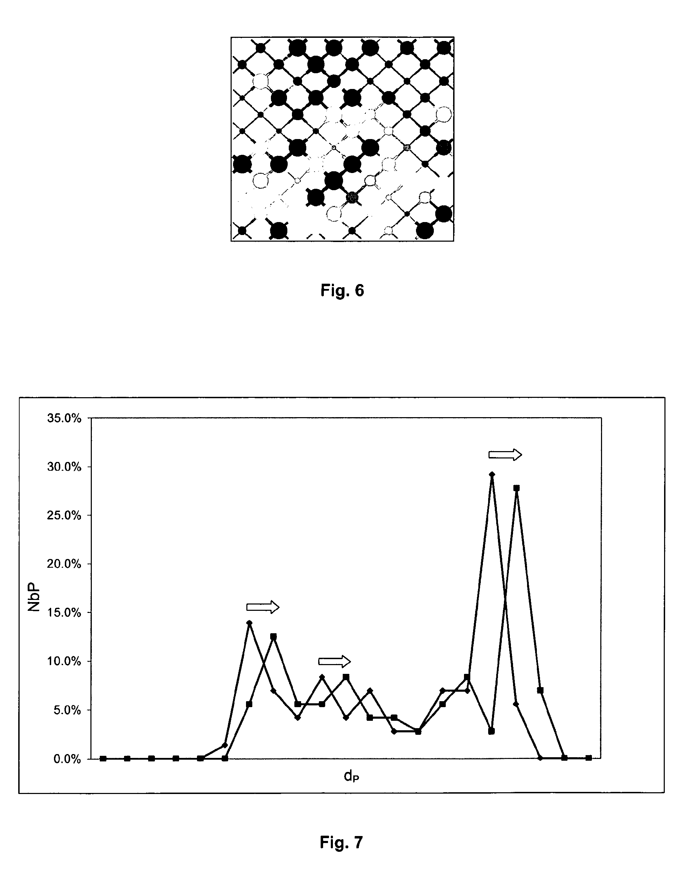

En appliquant le méthode selon l'invention on établit qu'il existe près de deux ordres de grandeur entre le coefficient réactif apparent des grands pores et celui des petits. Cette baisse du coefficient réactif apparent des grands pores se traduit par une accumulation du soluté dans ces volumes, ce qu'une carte de concentration permet aisément de vérifier (figure 6, où les fortes concentrations sont en noir, les ronds représentant les pores, et les traits reliant les pores représentant des canaux). Par conséquent, la précipitation, qui est proportionnelle au déséquilibre chimique, sera plus intense dans ces pores et, dans une moindre mesure, le long des chemins les reliant. Ceci se traduit par une chute plus prononcée de la porosité à la fin des périodes de première précipitation (comparer les figures 4 et 5). La dissolution reste, quant à elle, sensiblement uniforme. Il est possible de vérifier cette assertion rapidement à partir de la méthode en traçant la distribution de tailles de pores. Dans notre cas, celle-ci est quasiment translatée vers les grands pores, comme l'illustre la figure 7, où la courbe avec les losanges représente le nombre de pores (*NbP*) en fonction du diamètre $d_p$ des pores avant réaction, et la courbe avec les carrés représente le nombre de pores (*NbP*) en fonction du diamètre $d_p$ des pores après réaction.

En revanche, la modification de la partie précipitation bouleverse entièrement l'allure du cycle diagénétique (figure 5). En effet, les matières dissoutes dans les restrictions se déposent dans les pores ce qui conduit à une hausse très importante de la perméabilité. Ainsi, il est possible d'expliquer grâce à la méthode selon l'invention, comment on peut observer un cycle diagénétique totalement différent malgré des nombres adimensionnels moyens identiques.

En résumé, ces expériences montrent que pour le régime réactif choisi, nous avons, dans le cas où la structure initiale est homogène, une décroissance de perméabilité, alors que pour une certaine distribution hétérogène, ce même régime conduit à l'augmentation de celle-ci. En d'autres termes, de petites perturbations initiales au sein du milieu sont en mesure de conduire à de profondes hétérogénéités au cours de la diagenèse. En revanche, si la réaction est très lente, précipitation et dissolution deviennent réversibles et les courbes sont confondues comme pour la figure 3.

La méthode selon l'invention constitue donc un outil efficace et simple pour :

- modéliser physiquement les phénomènes de transport, de dissolution et de précipitation à l'échelle du pore,

- interpréter les mécanismes à l'aide des régimes hydrodynamique et réactionnel ainsi que des propriétés structurales du milieu,

- fournir des lois qui permettent un changement d'échelle entre le pore et la carotte puis, en utilisant les corrélations obtenues dans les simulateurs réservoir existants, entre la carotte et le réservoir

La méthode permet en outre d'effectuer une étude de sensibilité sur quelques paramètres clefs, tels que la distribution de taille de pores ou encore le rapport d'aspect, permettant ainsi d'étudier différents scénarios de diagenèse.

Enfin, la méthode a été décrite dans le cadre d'une détermination de la perméabilité et la porosité d'un milieu poreux. Néanmoins l'invention, par sa mise à jour de la structure du modèle du réseau poreux, s'applique à toutes propriétés pétrophysiques, telles que la pression capillaire et les perméabilités relatives.

**Revendications**

1. Méthode pour déterminer quantitativement une évolution de perméabilité et de porosité d'un milieu poreux au cours d'une diagenèse, ledit milieu poreux comportant un réseau de pores, **caractérisé en ce que** la méthode comporte les étapes suivantes :

   - on définit un scénario de diagenèse comportant un ensemble d'étapes de précipitation et de dissolution dans le milieu poreux, ainsi qu'une structure initiale du réseau de pores, au moyen de mesures physiques et d'observations dudit milieu ;
   - on construit une représentation du réseau de pores, au moyen d'un modèle PNM comportant un ensemble de noeuds de géométrie connue reliés par des canaux de géométrie connue ; puis, pour chaque étape dudit scénario de diagenèse, on réalise les étapes *a* à *d* suivantes :

     a. on détermine une concentration en ions aux parois de chaque noeud et canal ;
     b. on en déduit une variation de géométrie des noeuds et canaux dudit modèle PNM ;
     c. on détermine une perméabilité et une porosité dudit modèle PNM ainsi modifiées ;
     d. on réitère les étapes a à c jusqu'à ce que ladite étape soit achevée conformément au scénario de diagenèse ; et

     - on détermine une loi entre la perméabilité du milieu poreux et la porosité du milieu poreux au cours de la diagenèse.

2. Méthode selon la revendication 1, dans laquelle on construit la représentation du réseau de pores au moyen d'expériences d'invasion de mercure sur des carottes extraites dudit milieu poreux.

3. Méthode selon l'une des revendications précédentes, dans laquelle on détermine également à la fin de chaque étape dudit scénario de diagenèse d'autres propriétés pétrophysiques, telles que pression capillaire et perméabilités relatives.

4. Méthode selon l'une des revendications précédentes, dans laquelle on détermine la porosité au moyen de calculs de volumes connaissant la géométrie dudit modèle PNM.

5. Méthode selon l'une des revendications précédentes, dans laquelle on détermine la perméabilité selon la loi de Darcy.

6. Méthode pour déterminer l'emplacement potentiel d'un réservoir souterrain au sein d'un bassin sédimentaire constituant un milieu poreux, dans laquelle on détermine une loi entre la perméabilité du milieu poreux et la porosité du milieu poreux au cours d'un phénomène de diagenèse subit par le bassin au moyen de la méthode selon la revendication 1, et on étudie des écoulements de fluides au sein dudit bassin au moyen d'un simulateur de bassin renseigné par ladite loi.

7. Méthode pour améliorer une récupération d'hydrocarbures dans un réservoir souterrain constituant un milieu poreux, dans laquelle on caractérise des hétérogénéités dudit réservoir en déterminant une loi entre la perméabilité et la porosité du réservoir au cours d'un phénomène de diagenèse subit par le réservoir au moyen de la méthode selon la revendication 1, et on étudie des écoulements de fluides au sein dudit réservoir au moyen d'un simulateur de réservoir renseigné par ladite loi.

**Fig. 1**

**Fig. 2**

C

N

**Fig. 3**

**Fig. 4**

**Fig. 5**

**Fig. 6**

**Fig. 7**

**Europäisches Patentamt**
**European Patent Office**
**Office européen des brevets**

# RAPPORT DE RECHERCHE EUROPEENNE

Numéro de la demande

EP 09 29 0897

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | AHARONOV ET AL.: "transport properties and diagenesis in sedimentary rocks" GEOLOGY, vol. 25, no. 6, 1997, pages 547-550, XP008108413 * page 548 * ----- | 1-7 | INV. G01V11/00 ADD. G01N15/00 G01N15/08 G01N33/24 G06F17/50 |
| A | YOUSSEF ET AL.: "high-resolution CT and pore-network models" SPE/EAGE RESERVOIR CHARACTERIZATION AND SIMULATION CONFERENCE, 28 octobre 2007 (2007-10-28), - 30 octobre 2007 (2007-10-30) pages 280-291, XP008108412 abu dhabi * page 280, colonne 2, alinéa 1 * ----- | 1-7 | |
| A | OREN: "extending predictive capabilities to network models" SPE 388880 ANNUAL TECHNICAL CONFERENCE AND EXHIBITION, 5 octobre 1997 (1997-10-05), - 8 octobre 1997 (1997-10-08) pages 369-381, XP008108410 san antonio texas * abrégé * ----- | 1-7 | |
| A | GB 2 346 230 A (SCHLUMBERGER LTD [US]) 2 août 2000 (2000-08-02) * page 1, ligne 19 - ligne 20 * ----- | 1 | |
| A | US 2005/256643 A1 (BOITNOTT GREGORY N [US]) 17 novembre 2005 (2005-11-17) * alinéa [0026] * ----- -/-- | 1 | |

DOMAINES TECHNIQUES RECHERCHES (IPC)

G01N
G01V

Le présent rapport a été établi pour toutes les revendications

| Lieu de la recherche | Date d'achèvement de la recherche | Examinateur |
|---|---|---|
| La Haye | 3 juin 2010 | Hocquet, Alain |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

......................................................................

& : membre de la même famille, document correspondant

EPO FORM 1503 03.82 (P04C02)

Europäisches
Patentamt

European
Patent Office

Office européen
des brevets

## RAPPORT DE RECHERCHE EUROPEENNE

Numéro de la demande

EP 09 29 0897

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | LI L ET AL: "Upscaling geochemical reaction rates using pore-scale network modeling"<br>ADVANCES IN WATER RESOURCES, CML PUBLICATIONS, SOUTHAMPTON, GB,<br>vol. 29, no. 9,<br>1 septembre 2006 (2006-09-01), pages 1351-1370, XP025130323<br>ISSN: 0309-1708<br>[extrait le 2006-09-01]<br>* abrégé *<br>* page 1367, colonne 2, alinéa 8 - page 1368, colonne 1 *<br>----- | 1 | |
| A,D | CARMAN ET AL: "Fluid flow through granular beds"<br>CHEMICAL ENGINEERING RESEARCH AND DESIGN, PART A, INSTITUTION OF CHEMICAL ENGINEERS, XX,<br>vol. 75, 1 décembre 1997 (1997-12-01), pages S32-S48, XP025678629<br>ISSN: 0263-8762<br>[extrait le 1997-12-01]<br>* le document en entier *<br>----- | 1 | DOMAINES TECHNIQUES RECHERCHES (IPC) |
| A,D | LAROCHE C ET AL: "Two-Phase Flow Properties Prediction from Small-Scale Data Using Pore-Network Modeling"<br>TRANSPORT IN POROUS MEDIA, KLUWER ACADEMIC PUBLISHERS, DO,<br>vol. 61, no. 1,<br>1 octobre 2005 (2005-10-01), pages 77-91, XP019268837<br>ISSN: 1573-1634<br>* le document en entier *<br>-----<br>-/-- | 1 | |

Le présent rapport a été établi pour toutes les revendications

| Lieu de la recherche | Date d'achèvement de la recherche | Examinateur |
|---|---|---|
| La Haye | 3 juin 2010 | Hocquet, Alain |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 09 29 0897

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (IPC) |
| A,D | SHAPIRO ET AL.: "dispersion of a chemically reactive solute in a spatially periodic model of a porous medium" CHEMICAL ENGINEERING SCIENCE, vol. 43, no. 3, 1988, pages 551-571, XP002537714 GB * le document en entier * ----- | 1 | |
| A,D | BEKRI ET AL.: "dissolution of porous media" CHEMICAL ENGINEERING SCIENCE, vol. 50, no. 17, 1995, pages 2765-2791, XP002537715 gb * le document en entier * ----- | 1 | |
| | | | **DOMAINES TECHNIQUES RECHERCHES (IPC)** |

Le présent rapport a été établi pour toutes les revendications

| Lieu de la recherche | Date d'achèvement de la recherche | Examinateur |
|---|---|---|
| La Haye | 3 juin 2010 | Hocquet, Alain |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 09 29 0897

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-06-2010

| Document brevet cité au rapport de recherche | | Date de publication | Membre(s) de la famille de brevet(s) | | Date de publication |
|---|---|---|---|---|---|
| GB 2346230 | A | 02-08-2000 | US | 6088656 A | 11-07-2000 |
| US 2005256643 | A1 | 17-11-2005 | AUCUN | | |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Lucia, F. Jerry.** Carbonate Reservoir Characterization. Springer, 2007
- **Carman P. C.** Fluid flow through granular bed. *Trans. Inst. Chem. Eng. Lond.,* 1937, vol. 15, 150-166
- **Laroche, C. ; Vizika, O.** Two-phase flow properties prediction from small-scale data using pore-network modeling. *Transport in Porous Media,* 2005, vol. 61 (1), 77-91

- **Shapiro M. ; Brenner H.** Dispersion of a chemically reactive solute in a spatially model of a porous medium. *Chemical Engineering Science,* 1988, vol. 43, 551-571
- **Bekri S. ; Thovert J.-F. ; Adler P.M.** Dissolution of porous media. *Chem. Eng. Sci.,* 1995, vol. 50 (17), 2765-2791